(19) Europäisches Patentamt
European Patent Office
Office européen des brevets

(11) Veröffentlichungsnummer: **0 096 293**
**A1**

(12) # EUROPÄISCHE PATENTANMELDUNG

(21) Anmeldenummer: **83105147.9**

(22) Anmeldetag: **25.05.83**

(51) Int. Cl.³: **C 07 H 13/04**
C 07 H 15/04, C 07 H 3/04
A 61 K 31/70, A 61 K 39/015
A 61 K 39/00

(30) Priorität: **29.05.82 DE 3220426**

(43) Veröffentlichungstag der Anmeldung:
**21.12.83 Patentblatt 83/51**

(84) Benannte Vertragsstaaten:
**AT BE CH DE FR GB IT LI LU NL SE**

(71) Anmelder: **BEHRINGWERKE Aktiengesellschaft**
**Postfach 1140**
**D-3550 Marburg 1(DE)**

(72) Erfinder: **Hermentin, Peter, Dr.**
**Barfüssertor 30**
**D-3550 Marburg 1(DE)**

(74) Vertreter: **Meyer-Dulheuer, Karl-Hermann, Dr. et al,**
**HOECHST Aktiengesellschaft Zentrale Patentabteilung**
**Postfach 80 03 20**
**D-6230 Frankfurt/Main 80(DE)**

(54) **N-Acetylglucosaminderivate, Verfahren zu ihrer Herstellung und ihre Verwendung zur Bekämpfung der Malaria.**

(57) Es wird eine Verbindung der Formel 1 beschrieben,

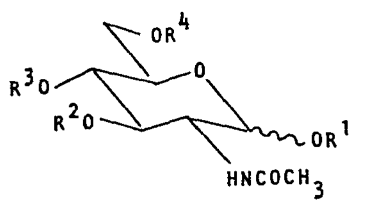

1

worin $R^1$ Wasserstoff, $C_1$ bis $C_8$-Alkanyl, -Alkenyl oder Alkinyl, jeweils verzweigt oder unverzweigt, oder ein alizyklischer oder aromatischer Kohlenwasserstoff-Rest, substituiert oder unsubstituiert, bevorzugt Benzyl oder substituiertes Benzyl oder ein Spacer, der zur Anknüpfung an Proteine oder an aminierte Matrizes geeignet oder an einen natürlichen oder synthetischen Träger gebunden ist, und $R^2$, $R^3$ und $R^4$ Wasserstoff oder alpha-L-Fucopyranosyl sind, wobei jedoch höchstens ein Rest alpha-L-Fucopyranosyl ist sowie ein Verfahren zu ihrer Herstellung und ihre Verwendung zur Bekämpfung der Malaria.

EP 0 096 293 A1

BEHRINGWERKE AKTIENGESELLSCHAFT HOE 82/B 009 Dr.HA/Bl.

# N-Acetylglucosaminderivate, Verfahren zu ihrer Herstellung und ihre Verwendung zur Bekämpfung der Malaria

Die Erfindung betrifft die Verwendung einer chemischen Verbindung der allgemeinen Formel I

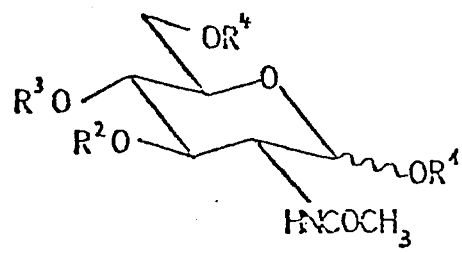

I

zur Chemotherapie und Chemoprophylaxe der Malaria sowie zur Herstellung eines Impfstoffes gegen die Malaria, wobei für Formel I gilt:

1.1. $R^1$ ist Wasserstoff oder

1.2. $R^1$ ist ein $C_1$ bis $C_8$-Alkanyl, -Alkenyl oder -Alkinyl, jeweils verzweigt oder unverzweigt, oder ein alizyklischer oder aromatischer Kohlenwasserstoff-Rest, substituiert oder unsubstituiert, bevorzugt Benzyl oder substituiertes Benzyl oder

1.3. ein Spacer, der zur Anknüpfung an Proteine oder an aminierte Matrizes geeignet ist oder

1.4. ein Spacer, gebunden an einen natürlichen oder synthetischen Träger, und

1.5. $R^2$, $R^3$ und $R^4$ sind Wasserstoff oder alpha-L-Fucopyranosyl, wobei jedoch höchstens ein Rest alpha-L-Fucopyranosyl ist.

2. Wenn $R^1$ ein Spacer ist, hat er die allgemeine Formel $-R-COR^5$, besitzt eine Länge von 0,3 nm bis 3 nm und kann die Atome C, H, O, N, S und P enthalten, wobei eine Reaktionsmöglichkeit mit den übrigen in der Verbindung der Formel I enthaltenen Gruppen ausgeschlossen ist.

3. Als Spacer ist $R^1$ bevorzugt
3.1. $-(CH_2)_nNHCO(CH_2)_mCOR^5$ oder
3.2. $-(CH_2)_nCONH(CH_2)_mCOR^5$ oder
3.3. $-(CH_2)_xCOR^5$, wobei
3.4. n = 1 bis 10, m = 1 bis 10,
3.5. x = 3 bis 17 und
3.6.1. $R^5$ -H oder -OH ist, oder
3.6.2. $R^5$ ist $C_1$-$C_8$-Alkanoxy, -Alkenoxy oder -Alkinoxy, jeweils verzweigt oder unverzweigt, O-Phenyl,bevorzugt durch induktive Atome oder Gruppen, wie $NO_2$, substituiert, O-Benzyl, substituiert oder unsubstituiert, oder
3.6.3. -NH, $-NHNH_2$ oder $-N_3$.

4. $R^1$ ist bevorzugt $-(CH_2)_nNHCO(CH_2)_mCOR^5$ mit $R^5$ = Alkoxy und n = 2 und m = 4 oder 7.

5. $R^1$ ist ferner bevorzugt $-(CH_2)_xCOR^5$ mit $R^5$ = Alkoxy und x = 3 bis 10 und besonders bevorzugt mit x = 8.

6. Wenn $R^1$ Spacer und Träger bedeutet, ist $R^5$ ein löslicher oder unlöslicher, natürlicher oder synthetischer Träger, bevorzugt ein Protein oder ein Aminogruppen-tragendes synthetisches oder natürliches Polymer, bevorzugt Humanalbumin, Kollagen oder aminiertes Kieselgel.

7. Die glycosidische Bindung zum Spacer ist bevorzugt beta.

8. Die Erfindung betrifft die Verwendung einer chemischen Verbindung der Formel I

8.1. zur Chemotherapie und Chemoprophylaxe der Malaria sowie

8.2 zur Herstellung eines Impfstoffes gegen die Malaria.

9. Die Erfindung betrifft weiterhin eine chemische Verbindung der Formel I,

9.1. wo $R^1$ Wasserstoff ist

9.2. und $R^2$, $R^3$ und $R^4$ die unter 1.5 genannte Bedeutung haben

9.3. wobei jedoch die Verbindung ausgenommen ist, in der $R^3$ alpha- L-fucopyranosyl ist und $R^1$, $R^2$ und $R^4$ Wasserstoff sind,

10. sowie ein Verfahren zu ihrer Herstellung.

11. Die Erfindung betrifft weiterhin eine Verbindung der Formel I,

11.1. wo $R^1$ die unter 1.2. genannte Bedeutung hat

11.2. und $R^2$, $R^3$ und $R^4$ die unter 1.5. genannte Bedeutung haben

12. sowie ein Verfahren zu ihrer Herstellung.

13. Die Erfindung betrifft weiterhin eine Verbindung der Formel I,

13.1. wo $R^1$ wie in 1.2. ist, und

13.2.1. $R^4$ 2,3,4-Tri-O-benzyl-alpha-L-fucopyranosyl ist und $R^2$ und $R^3$ zusammen Alkyliden, bevorzugt Isopropyliden, sind,

13.2.2. oder $R^2$ 2,3,4-Tri-O-benzyl-alpha-L-fucopyranosyl ist und $R^3$ und $R^4$ zusammen Alkyliden, bevorzugt Benzyliden, sind,

13.2.3. oder $R^3$ 2,3,4-Tri-O-benzyl-alpha-L-fucopyranosyl ist und $R^2$ Benzyl, Benzyloxymethyl, Methyloxymethyl oder Allyl und $R^4$ Benzyl,Benzyloxymethyl oder Acyl, bevorzugt Acetyl, ist,

13.2.4. wobei jedoch für eine Verbindung gemäß 13.2.3. die Verbindung ausgenommen ist, in der $R^2$ Allyl oder Benzyl und $R^4$ Benzyl ist, sofern $R^1$ alpha-Benzyl ist,

14. sowie ein Verfahren zu ihrer Herstellung.

15. Die Erfindung betrifft weiterhin eine Verbindung der Formel I,

15.1.1. in der $R^1$ ein Spacer gemäß 1.3. und 2. ist,

15.1.2. bevorzugt ein Spacer gemäß 3.1. bis 3.6.2,

15.2. und $R^2$, $R^3$ und $R^4$

15.2.1. die unter 1.5.

15.2.2. oder die unter 13.2.1.

15.2.3. oder die unter 13.2.2.

15.2.4. oder die unter 13.2.3.

genannte Bedeutung haben,

15.3.1. wobei jedoch die Verbindung ausgenommen ist, in der der Spacer wie in 3.3. ist

15.3.2. mit $R^5$ wie in 3.6.1. oder 3.6.2.,

15.3.3. sofern die glykosidische Bindung zum Spacer beta ist,

16. sowie ein Verfahren zu ihrer Herstellung.

17. Die Erfindung betrifft weiterhin eine Verbindung der Formel I,

17.1. in der $R^1$ - gemäß 1.4.- ein Spacer, gebunden an einen natürlichen oder synthetischen Träger, ist

17.2. und $R^2$, $R^3$ und $R^4$ die unter 1.5. genannte Bedeutung haben,

17.3.1. wobei jedoch die Verbindung ausgenommen ist, in der ein Spacer gemäß 3.3. vorliegt,

17.3.2. sofern die glycosidische Bindung zum Spacer beta ist,

18. sowie ein Verfahren zu ihrer Herstellung.

Als idealer Impfstoff gegen die Malaria wird heute ein isoliertes, gereinigtes, nicht-toxisches, lösliches Parasiten-Antigen erachtet, das stabil, präservierbar und immunogen per se ist, d.h. bei der Applikation kein Adjuvans benötigt (R.S.Desowitz und L.H.Miller, Bull. WHO (1980) 58, 897-908). Einen solchen Impfstoff gibt es nicht.

Es bestand daher die Aufgabe, Antigene des Malaria-Erregers P.falciparum zu isolieren, zu reinigen und zu charakterisieren und auf ihre mögliche Eignung als Malaria-Impfstoff zu testen.

Es ist bekannt, daß sich die Isolierung von Proteinen aus einem Proteingemisch durch Affinitätschromatographie stark vereinfachen läßt. In Analogie hierzu lassen sich Antigene durch Immunadsorbentien, an welche entsprechende Antikörper oder Lektine gebunden sind, und Antikörper durch Immunadsorbentien, an welche entsprechende Antigene gebunden sind, isolieren. In dieser Hinsicht ist neuerdings der Zugang zu monoklonalen Antikörpern, etwa durch Zellfusion (G.Köhler und C.Milstein, Nature (1975), 256, 495-497), als auch der Zugang zu synthetischen Kohlenhydrat-Antigenen, die mittels eines geeigneten Spacers an ein Trägerprotein oder eine feste Matrix gebunden werden können (R.U.Lemieux, D.R.Bundle und D.A.Baker, DOS 26 30 596 (1977); R.U.Lemieux, Chem.Soc.Rev. (1978) 7, 423-452), von besonderem Interesse.

Überraschenderweise wurde nun gefunden, daß Verbindungen der Formel I zur selektiven Isolierung von Rezeptoren des Malaria-Erregers Plasmodium falciparum geeignet sind, besonders wenn diese mittels eines Spacers an eine feste Matrix gebunden sind.

Weiterhin wurde gefunden, daß Verbindungen der Formel I zur Chemotherapie und Chemoprophylaxe der Malaria verwendet werden können.

Vanderberg et al. (M.M. Weiss, J.D. Oppenheim und J.P. Vanderberg, Exp. Parasitol. (1981) 51, 400-407) haben gefunden, daß der Befall von Humanerythrozyten durch *P. falciparum*-Merozoiten in synchroner in vitro-Kultur durch Zusatz von N-Acetyl-D-glucosamin (DGlcNAc), D-Glucosamin-hydrochlorid ($DGlcNH_3Cl$) und L-Fucose gehemmt wird. Sie haben ferner gefunden, daß das intraerythrozytäre Wachstum der Malaria-Parasiten durch $DGlcNH_3Cl$ oder L-Fucose, nicht aber durch N-Acetyl-D-Glucosamin gehemmt wird. Daraus haben sie gefolgert, daß $DGlcNH_3Cl$ und L-Fucose für den Malaria-Erreger toxisch sind.

Die Autoren haben vermutet, daß D-GlcNAc als spezifischer Penetrations-Inhibitor wirkt, indem es einen für den Invasions-Vorgang essentiellen Rezeptor blockiert.

Sie haben ferner vermutet, daß DGlcNAc eine wichtige Komponente des Glycoprotein-Rezeptors darstellt, der das Erkennen oder die Penetration von *P. falciparum*-Merozoiten in Humanerythrozyten vermittelt, während $D-GlcNH_3Cl$ und L-Fucose toxisch wirken. Überraschenderweise wurde nun gefunden, daß D-GlcNAc - entgegen der Befunde von Vanderberg et al. - für das intraerythrozytäre Parasiten-Wachstum toxisch ist, während L-Fucose - wiederum entgegen der Befunde von Vanderberg et al. - nicht toxisch ist. Ferner wurde gefunden, daß das zu DGlcNAc korrespondierende alpha-oder beta-Methylglycosid, Methyl-2-acetamido-2-desoxy-alpha oder beta-D-glucopyranosid ($\alpha$-oder $\beta$-DGlcNAcOMe), für das intraerythrozytäre Wachstum von

*P. falciparum* nicht toxisch wirkt, jedoch im Vergleich zu anderen Methylglycosiden, wie z. B. Methyl-α oder β-D-glucopyranosid, Methyl-α oder β-D-galactopyranosid, Methyl-α oder β-L-fucopyranosid oder Methyl-α-D-mannopyranosid, eine stark erhöhte invasionsinhibierende Wirkung besitzt.

Weiter wurde überraschenderweise gefunden, daß eine Verbindung der Formel I, in der L-Fucose in alphaglycosidischer Verknüpfung an N-Acetyl-D-glucosamin gebunden ist, die Inhibitionswirkung des DGlcNAc für den Befall von Humanerythrozyten durch *P.falciparum* - Merozoiten in synchroner in vitro-Kultur erhöht.

19.1. Gegenstand der Erfindung ist daher die Verwendung einer Verbindung der allgemeinen Formel I, worin $R^1$ bis $R^4$ die unter 1.1. bis 6. angegebene Bedeutung haben,

19.1.1. zur Chemotherapie und Chemoprophylaxe der Malaria sowie

19.1.2. zur Herstellung eines Impfstoffes gegen die Malaria.

19.2.1. Gegenstand der Erfindung ist weiterhin eine Verbindung der allgemeinen Formel I, in der $R^1$ bis $R^4$

19.2.1. die unter 9.1. und 9.2. genannte Bedeutung mit der unter 9.3. gemachten Einschränkung oder

19.2.2. die unter 11.1. und 11.2. genannte Bedeutung haben.

19.3. Gegenstand der Erfindung ist weiterhin eine Verbindung der allgemeinen Formel I, in der $R^1$ bis $R^4$ die unter 11.1. und 11.2. genannte Bedeutung haben.

19.4. Gegenstand der Erfindung ist weiterhin eine Verbindung der allgemeinen Formel I,

19.4.1. in der $R^1$ wie in 1.2. ist

19.4.2. und $R^2$ bis $R^4$ die in 13.2.1. oder 13.2.2. oder 13.2.3. genannte Bedeutung haben,

19.4.3. wobei jedoch für eine Verbindung gemäß 13.2.3. die in 13.2.4. gemachte Einschränkung gilt.

19.5. Gegenstand der Erfindung ist weiterhin eine Verbindung der allgemeinen Formel I,

19.5.1.1. in der $R^1$ ein Spacer gemäß 15.1.1.,

19.5.1.2. bevorzugt ein Spacer gemäß 15.1.2., ist,

19.5.2. und $R^2$ bis $R^4$ die unter 1.5. oder 13.2.1. oder 13.2.2. oder 13.2.3. genannte Bedeutung haben,

19.5.3. wobei jedoch die Verbindung ausgenommen ist,

19.5.3.1. in der $R^2$ bis $R^4$ wie in 1.5. sind

19.5.3.2.1. sofern der Spacer wie in 3.3. ist,

19.5.3.2.2. mit $R^5$ wie in 3.6.1. oder 3.6.2.,

19.5.3.2.3. und sofern die glycosidische Bindung zum Spacer beta ist.

19.6. Gegenstand der Erfindung ist weiterhin eine Verbindung der allgemeinen Formel I,

19.6.1. in der $R^1$ - gemäß 1.4.- ein Spacer, gebunden an einen natürlichen oder synthetischen Träger, ist,

19.6.2. und $R^2$, $R^3$ und $R^4$ die unter 1.5. genannte Bedeutung haben,

19.6.3.1. wobei jedoch die Verbindung ausgenommen ist, in der der Spacer wie in 3.3. ist

19.6.3.2. mit $R^5$ wie in 6.,

19.6.3.3. sofern die glycosidische Bindung zum Spacer beta ist.

20. Gegenstand der Erfindung ist weiterhin ein Verfahren zur Herstellung einer unter Punkt 19.2., 19.3., 19.4.; 19.5. oder 19.6. genannten Verbindung der allgemeinen Formel I.

Verfahren zur Herstellung einer Verbindung der Formel I

21. Das erfindungsgemäße Verfahren zur Herstellung einer chemischen Verbindung der Formel I ist dadurch gekennzeichnet, daß man

21.1. eine Verbindung der allgemeinen Formel II

$$HO(CH_2)_nNHCO(CH_2)_mCOR^5 ,$$

wobei n und m die unter 3.4. und $R^5$ die unter 3.6.2. gegebene Bedeutung haben, durch Umsetzung eines Aminoalkohols, beispielsweise 2-Aminoethanol, mit einem Dicarbonsäure-Monoester, beispielsweise Adipinsäuremonomethylester, in Gegenwart von Dicyclohexylcarbodiimid in trockenem Dimethylformamid synthetisiert oder

21.2. eine Verbindung der allgemeinen Formel III

$$HO(CH_2)_nCONH(CH_2)_mCOR^5 ,$$

wobei n und m und $R^5$ die unter 21.1. gegebene Bedeutung haben, beispielsweise in Analogie zu 21.1. durch Umsetzung einer ω-Hydroxycarbonsäure, beispielsweise Glycolsäure, mit einem ω-Aminocarbonsäureester, beispielsweise 4-Aminobuttersäureethylester,synthetisiert oder

21.3. eine Verbindung der allgemeinen Formel IV

$$HO(CH_2)_xCOR^5 ,$$

wobei x und $R^5$ die unter 3.5. beziehungsweise 3.6.2. gegebene Bedeutung haben, in an sich bekannter Weise (R.U.Lemieux, D.R. Bundle und D.A.Baker, J. Am. Chem. Soc. 97, (1975), 4076-4083) synthetisiert und

22.1. eine Verbindung der Formel II, III oder IV in an sich bekannter Weise(R.U.Lemieux, D.R.Bundle und D.A.Baker, J. Am. Chem. Soc. 97, (1975), 4076-4083) mit 2-Acetamido-3,4,6-tri-O-acyl (bevorzugt Acetyl oder Benzoyl)-2-desoxy-alpha-D-glucopyra-

nosylbromid oder -chlorid zu einer Verbindung der Formel I umsetzt, in der

22.1.1. $R^2$, $R^3$ und $R^4$ Acyl, bevorzugt Acetyl oder Benzoyl sind

22.1.2. und $R^1$ die unter 3. bis 3.5. gegebene Bedeutung hat mit $R^5$ wie in 3.6.2. und

22.1.3. die glycosidische Bindung zum Spacer beta ist,

22.1.4 wobei für die Umsetzung einer Verbindung der Formel IV die unter 15.3.1. und 15.3.2. gemachte Einschränkung gilt, oder

23. eine Verbindung der Formel II, III oder IV in an sich bekannter Weise (Europäische Patentanmeldung Nr. 00 44 188) mit 3,4,6-Tri-O-acyl (bevorzugt Acetyl oder Benzoyl)-2-azido-2-desoxy-ß-D-glucopyranosylchlorid zu einer Verbindung der Formel V umsetzt,

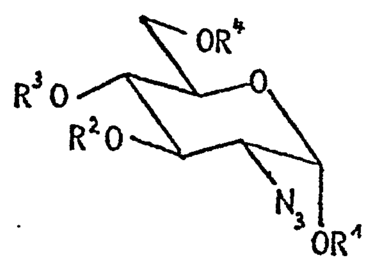

V

23.1.1. in der $R^1$ die unter 3. bis 3.5. angegebene Bedeutung hat wobei $R^5$ die unter 3.6.2. gegebene Bedeutung besitzt,

23.1.2. $R^2$, $R^3$ und $R^4$ wie unter 22.1.1. sind, und

23.1.3. die glycosidische Bindung zum Spacer alpha ist, und in an sich bekannter Weise

23.2.1. die 2-Azido-Gruppe zur Amino-Gruppe reduziert, bevorzugt durch katalytische Hydrierung oder durch Reduktion mit Schwefelwasserstoff in Pyridin/Triethylamin, und in an sich bekannter Weise,

23.2.2. bevorzugt mittels Acetanhydrid in trockenem Methanol, acetyliert,

23.3. wobei eine Verbindung der Formel I entsteht, in der

23.3.1. $R^1$ bis $R^5$ die unter 23.1.1. und 23.1.2. angegebenen Bedeutungen haben und

23.3.2. die glycosidische Bindung zum Spacer alpha ist, oder

24. den unter 22.1. genannten Halogeno-Zucker in an sich bekannter Weise mit einem Alkohol der allgemeinen Formel $HOR^1$, wobei $R^1$ die in 1.2. genannte Bedeutung hat,

25.1. zu einer Verbindung der Formel I umsetzt, in der

25.2. $R^2$, $R^3$ und $R^4$ wie unter 22.1.1. sind,

25.3. $R^1$ die in 1.2. genannte Bedeutung hat und

25.4. die glycosidische Bindung beta ist oder

26.1. den unter 23. genannten Halogeno-Zucker in an sich bekannter Weise mit einem Alkohol der allgemeinen Formel $HOR^1$ umsetzt, in der $R^1$ die unter 1.2. angegebene Bedeutung hat, wodurch eine Verbindung der allgemeinen Formel V erhalten wird, in der

26.2. $R^2$, $R^3$ und $R^4$ wie unter 22.1.1. sind,

26.3. $R^1$ die in 1.2. genannte Bedeutung hat und

26.4. die glycosidische Bindung alpha ist, welche

27.1. in Analogie zu 23.2.1. und 23.2.2. in eine Verbindung der allgemeinen Formel I überführt wird,

27.1.1. in der $R^1$ wie in 1.2. ist und $R^2$, $R^3$ und $R^4$ die in 22.1.1. angegebene Bedeutung haben und

27.1.2. die glycosidische Bindung zum Spacer alpha ist und

28. die nach 22.1., 23.3., 25.1.- 4. und 27.1.gewonnene Verbindung der Formel I in an sich bekannter Weise, bevorzugt mittels Natriummethylat in trockenem Methanol,

28.1. in eine Verbindung der Formel I überführt, in der

28.1.1. $R^2$, $R^3$ und $R^4$ Wasserstoff sind, und

28.1.2. $R^1$ die unter 1.2., 1.3. und 3. bis 3.5. genannte Bedeutung besitzt, wobei $R^5$ wie unter 3.6.2. ist,

29. welche man in an sich bekannter Weise zu einer Verbindung der Formel I umsetzt, in der

29.1. $R^1$ die unter 28.1.2. genannte Bedeutung besitzt,

29.2. $R^4$ Wasserstoff ist, und

29.3. $R^2$ und $R^3$, zusammen mit dem Brücken-C-Atom, Alkyliden, bevorzugt Isopropyliden, sind, oder

30. zu einer Verbindung der Formel I umsetzt, wo

30.1. $R^1$ wie unter 28.1.2. und

30.2. $R^2$ Wasserstoff ist, und

30.3. $R^3$ und $R^4$, zusammen mit dem Brücken-C-Atom, Benzyliden oder Alkyliden sind, oder

31. zu einer Verbindung gemäß 30. umsetzt, welche

32. über eine Verbindung der Formel I,

32.1. in der $R^1$ die unter 28.1.2., und

32.2. $R^3$ und $R^4$, zusammen mit dem Brücken-C-Atom, die in 30.3. gegebene Bedeutung haben, und

32. $R^2$ Benzyl, Benzyloxymethyl, Methyloxymethyl oder Allyl ist, und

33. über eine Verbindung der Formel I,

33.1. in der $R^1$ die unter 28.1.2., und

33.2. $R^2$ die unter 32.3. genannte Bedeutung besitzt, und

33.3. $R^3$ und $R^4$ Wasserstoff sind,

34. in eine Verbindung der Formel I überführt,

34.1. in der $R^1$ die unter 28.1.2., und

34.2. $R^2$ die in 32.3. genannte Bedeutung besitzt,

34.3. $R^3$ Wasserstoff und

34.4. $R^4$ Benzyl oder Benzyloxymethyl oder Acyl, bevorzugt Acetyl, ist, und

35. man die in 29., 30. oder 34. erhaltene Verbindung in an sich bekannter Weise

35.1. mit 2,3,4-Tri-O-benzyl-alpha-L-fucopyranosylbromid oder

35.2. mit 1-O-(N-methyl)-acetimidyl-2,3,4-tri-O-benzyl-ß-L-fucopyranose

36. zu einer Verbindung der Formel I,umsetzt,

36.1. wo $R^1$ wie in 28.1.2. ist, und

36.2. $R^2$, $R^3$ und $R^4$ wie in 13.2.1. oder 13.2.2. oder 13.2.3.sind, worauf in an sich bekannter Weise

37. die Schutzgruppen einer Verbindung der Formel I mit $R^1$ wie in 28.1.2.und mit $R^2$ bis $R^4$ wie in 13.2.1. in beliebiger Reihenfolge durch Hydrolyse, bevorzugt durch verdünnte Trifluoressigsäure, und Hydrogenolyse, bevorzugt mittels Palladium auf Kohle, oder

38. die Schutzgruppen einer Verbindung der Formel I mit $R^1$ wie in 28.1.2. und mit $R^2$ bis $R^4$ wie in 13.2.2.

38.1. entweder hydrogenolytisch, sofern $R^3$ und $R^4$ zusammen Benzyliden sind

38.2. oder hydrolytisch und hydrogenolytisch, sofern $R^3$ und $R^4$ zusammen Alkyliden sind, oder

39. die Schutzgruppen einer Verbindung der Formel I mit $R^1$ wie in 28.1.2. und mit $R^2$ bis $R^4$ wie in 13.2.3.

39.1. entweder hydrolytisch, sofern $R^2$ und $R^4$ Benzyl oder Benzyloxymethyl sind,

39.2. oder mittels Titaniumtetrachlorid und hydrogenolytisch, sofern $R^2$ Methyloxymethyl und $R^4$ Benzyl oder Benzyloxymethyl ist,

39.3. oder mittels Tris-(triphenylphosphin)-Rhodium-(I)-chlorid und einer Mischung aus Quecksilberbromid und -oxid und hydrogenolytisch, sofern $R^2$ Allyl und $R^4$ Benzyl oder Benzyloxymethyl ist,

39.4. oder mittels Natriummethylat in trockenem Methanol und hydrogenolytisch, sofern $R^4$ Acyl, bevorzugt Acetyl, und $R^2$ Benzyl oder Benzyloxymethyl ist,

39.5. oder in einer Kombination der Reaktionen gemäß 39.4. und 39.2., sofern $R^4$ Acyl, bevorzugt Acetyl, und $R^2$ Methyloxymethyl ist,

39.6. oder in einer Kombination der Reaktionen gemäß 39.4. und 39.3., sofern $R^4$ Acyl, bevorzugt Acetyl, und $R^2$ Allyl ist, abgespalten werden,

40. wobei eine Verbindung der Formel I erhalten wird, in der

40.1. $R^2$, $R^3$ und $R^4$ Wasserstoff oder alpha-L-Fucopyranosyl sind,

40.2. wobei jedoch höchstens ein Rest alpha-L-Fucopyranosyl ist,

40.3. und $R^1$ die unter 28.1.2. genannte Bedeutung besitzt,

40.4.1. jedoch in dem Falle, wo $R^1$ vor der Schutzgruppenabspaltung Benzyl war, $R^1$ jetzt Wasserstoff ist,

40.4.2. wobei jedoch die Verbindung ausgenommen ist, in der $R^3$ alpha-L-Fucopyranosyl ist und $R^1$, $R^2$ und $R^4$ Wasserstoff sind

41. und eine Verbindung gemäß Punkt 40., für die die Einschränkung gilt, daß $R^1$ nicht die unter 1.2. genannte Bedeutung besitzt,

42. in an sich bekannter Weise in eine Verbindung der allgemeinen Formel I überführt wird,

42.1. in der $R^2$, $R^3$ und $R^4$ die unter 40.1. genannte Bedeutung haben und der in 40.2. genannten Einschränkung unterliegen,

42.2. und $R^1$ ein unter Punkt 2. genannter,

42.3. bevorzugt ein gemäß Punkt 3. bis 3.5. klassifizierter Spacer ist,

42.4. bei dem $R^5$ -OH, $-NHNH_2$ oder-$N_3$ ist

43. und schließlich eine Verbindung gemäß Punkt 42. in an sich bekannter Weise (R.U.Lemieux, D.R. Bundle und D.A.Baker, DE-OS 26 30 596 (1977)) an einen löslichen oder unlöslichen Träger, welcher freie Aminogruppen trägt, angekuppelt oder angeknüpft wird.

In Frage kommende Träger sind zum Beispiel Peptide oder Proteine, bevorzugt Rinderserumalbumin oder Humanalbumin, rote oder weiße Blutkörperchen, aminiertes Kieselgel, Aminogruppen tragende Kunststoffe oder Aminogruppen tragende Oligo- oder Polysaccharide.

Eine auf diese Weise erhaltene Verbindung kann, je nach Beschaffenheit, als humanverträgliches Makromolekül oder als Antigen oder als Immunadsorbens dienen.

0096293

Verwendung einer Verbindung der Formel I zur Chemotherapie und Chemoprophylaxe der Malaria

Zur Chemotherapie und Chemoprophylaxe der Malaria wird eine humanverträgliche Verbindung der Formel I, worin die Reste $R^1$ bis $R^4$ bzw. $R^5$ die unter 1.1. bis 6. angegebene Bedeutung haben, oral oder parenteral, ein oder mehrmals, gegebenenfalls in Verbindung mit einem humanverträglichen Hilfs-und/oder Zusatzstoff, in einer wirksamen Menge von zum Beispiel 0,1 µg bis 100 mg Wirkstoff je kg Körpergewicht verabreicht.

Verwendung einer Verbindung der Formel I zur Herstellung einer Malaria-Vaccine

Zur Herstellung eines Impfstoffes gegen die Malaria werden intakte Merozoiten oder ein Merozoiten-Lysat mit einer Verbindung der Formel I, worin $R^1$ ein Spacer ist, der an einen festen Träger, bevorzugt an eine aminierte Matrix, gebunden ist, und $R^2$ bis $R^4$ die in 1.5. genannte Bedeutung haben, inkubiert. Die inkubierten intakten Merozoiten werden unter gepufferten physiologischen Bedingungen (A.Kilejian, Proc. Natl. Acad. Sci. USA (1980), 77, 3695-3699) lysiert.

Der so behandelte Träger wird gewaschen und nachfolgend, bevorzugt über eine Säule, durch chaotrope Änderung oder bei einem pH zwischen 2,5 und 5 oder zwischen 8 und 11 oder durch Zusatz von N-Acetylglucosamin oder α-DGlcNAcOMe eluiert.
Das auf diese Weise gewonnene Produkt, das in an sich bekannter Weise gereinigt werden kann, wird als Impfstoff zur aktiven Immunisierung gegen Malaria verabreicht.

0096293

1. Verwendung einer chemischen Verbindung der allgemeinen Formel I

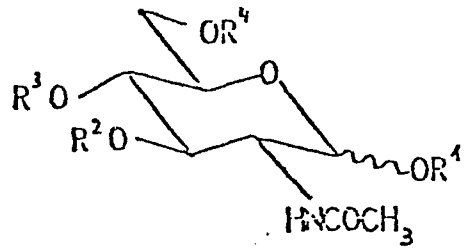

I

zur Bekämpfung der Malaria wobei für Formel I gilt:

1.1. $R^1$ ist Wasserstoff oder

1.2. $R^1$ ist ein $C_1$ bis $C_8$-Alkanyl, -Alkenyl oder Alkinyl, jeweils verzweigt oder unverzeigt, oder ein alizyklischer oder aromatischer Kohlenwasserstoff-Rest, substituiert oder unsubstituiert, bevorzugt Benzyl oder substituiertes Benzyl, oder

1.3. ein Spacer, der zur Anknüpfung an Proteine oder an aminierte Matrizes geeignet ist, oder

1.4. ein Spacer, gebunden an einen natürlichen oder synthetischen Träger, und

1.5. $R^2$, $R^3$ und $R^4$ sind Wasserstoff oder alpha-L-Fucopyranosyl, wobei jedoch höchstens ein Rest alpha-L-Fucopyranosyl ist.

2. Verwendung einer Verbindung nach Anspruch 1, dadurch gekennzeichnet, daß der Spacer die allgemeine Formel $-R-COR^5$ und eine Länge von 0,3 nm bis 3 nm besitzt und die Atome C, H, O, N, S und P enthalten kann, wobei eine Reaktionsmöglichkeit mit den übrigen in der Verbindung der Formel I enthaltenen Gruppen ausgeschlossen ist.

3. Verwendung einer Verbindung nach Anspruch 1, dadurch gekennzeichnet, daß der Spacer $-(CH_2)_nNHCO(CH_2)_mCOR^5$ ist, wobei n = 1 bis 10, m = 1 bis 10, und $R^5$ -H, -OH, $C_1$-$C_8$-Alkanoxy, -Alkenoxy oder -Alkinoxy, jeweils verzweigt oder unverzeigt, O-Phenyl, bevorzugt durch induktive Atome oder Gruppen, wie $-NO_2$, substituiert, O-Benzyl, substituiert oder unsubstituiert, $-NH_2$, $-NHNH_2$ oder $-N_3$ ist.

4. Verwendung einer Verbindung nach Anspruch 1, dadurch gekennzeichnet, daß der Spacer $-(CH_2)_nCONH(CH_2)_mCOR^5$ ist, wobei n = 1 bis 10, m = 1 bis 10, und $R^5$ -H, -OH, $C_1$-$C_8$-Alkanoxy, -Alkenoxy oder -Alkinoxy, jeweils verzweigt oder unverzweigt, O-Phenyl, bevorzugt durch induktive Atome oder Gruppen, wie $-NO_2$, substituiert, O-Benzyl, substituiert oder unsubstituiert, $-NH_2$, $-NHNH_2$ oder $-N_3$ ist.

5. Verwendung einer Verbindung nach Anspruch 1, dadurch gekennzeichnet, daß der Spacer $-(CH_2)_xCOR^5$ ist, wobei x = 3 bis 17 und $R^5$ -H, -OH, $C_1$-$C_8$-Alkanoxy, -Alkenoxy oder -Alkinoxy, jeweils verzweigt oder unverzweigt, O-Phenyl, bevorzugt durch induktive Atome oder Gruppen, wie $-NO_2$, substituiert, O-Benzyl, substituiert oder unsubstituiert, $-NH_2$, $-NHNH_2$ oder $-N_3$ ist.

6. Verwendung einer Verbindung nach Anspruch 1, dadurch gekennzeichnet, daß der Spacer $-(CH_2)_nNHCO(CH_2)_m COR^5$ oder $-(CH_2)_nCONH(CH_2)_mCOR^5$ mit n = 2, m = 4 oder 7 und $R^5$ -H, -OH, $C_1$-$C_8$-Alkoxy verzweigt oder unverzweigt, O-Phenyl, bevorzugt durch induktive Atome oder Gruppen, wie $-NO_2$, substituiert, O-Benzyl, substituiert oder unsubstituiert, $-NH_2$, $-NHNH_2$ oder $-N_3$ ist.

7. Verwendung einer Verbindung nach Anspruch 1, dadurch gekennzeichnet, daß der Spacer $-(CH_2)_xCOR^5$ mit $x = 3$ bis 10 und $R^5$ -H, -OH, $C_1$-$C_8$-Alkoxy, verzweigt oder unvezweigt, O-Phenyl, bevorzugt durch induktive Atome oder Gruppen, wie $-NO_2$, substituiert, O-Benzyl, substituiert oder unsubstituiert, $-NH_2$, $NHNH_2$ oer $-N_3$ ist.

8. Verwendung einer Verbindung nach Anspruch 1, dadurch gekennzeichnet, daß der Spacer $-(CH_2)_xCOR^5$ mit $x = 8$ und $R^5$ -H, -OH, $C_1$-$C_8$-Alkoxy, verzweigt oder unverzweigt, O-Phenyl, bevorzugt durch induktive Atome oder Gruppen, wie $-NO_2$, substituiert, O-Benzyl, substituiert oder unsubstituiert, $-NH_2$, $-NHNH_2$ oder $-N_3$ ist.

9. Verwendung einer Verbindung nach Anspruch 1, dadurch gekennzeichnet, daß wenn $R^1$ Spacer und Träger bedeu - tet, $R^5$ ein löslicher natürlicher Träger, bevorzugt ein Protein ist.

10. Verwendung einer Verbindung nach Anspruch 1, dadurch gekennzeichnet, daß wenn $R^1$ Spacer und Träger bedeutet, $R^5$ ein löslicher natürlicher Träger, bevorzugt Humanalbumin, Kollagen oder Rinderserumalbumin ist.

11. Verwendung einer Verbindung nach Anspruch 1, dadurch gekennzeichnet, daß wenn $R^1$ Spacer und Träger bedeutet, $R^5$ ein unlöslicher natürlicher oder synthetischer Träger, bevorzugt ein Aminogruppen tragendes synthetisches oder natürliches Polymer ist.

12. Verwendung einer Verbindung nach Anspruch 1,dadurch gekennzeichnet, daß wenn $R^1$ Spacer und Träger bedeutet, $R^5$ aminiertes Kieselgel ist.

13. Verwendung einer Verbindung nach Anspruch 1, dadurch gekennzeichnet, daß die glycosidische Bindung zu $R^1$ beta ist.

14. Verwendung einer Verbindung nach Anspruch 1, dadurch gekennzeichnet, daß die glycosidische Bindung zu $R^1$ alpha ist.

15. Verwendung einer Verbindung nach einem der Ansprüche 1 bis 10 oder 13 oder 14 zur Chemotherapie und Chemoprophylaxe der Malaria.

16. Verwendung einer Verbindung nach einem der Ansprüche 1 bis 14 zur Herstellung eines Impfstoffes gegen die Malaria.

17. Verwendung einer Verbindung nach einem der Ansprüche 11 bis 14 in einem Verfahren zur Herstellung eines Impfstoffes gegen die Malaria, dadurch gekennzeichnet, daß man eine solche Verbindung in Suspension mit Merozoiten zusammenbringt, die an die Verbindung gebundenen Merozoiten lysiert und die ungelöste Phase abtrennt, wäscht und die an die Verbindung gebundenen Merozoit-Bestandteile durch chaotrope oder pH-Änderung oder durch Zusatz von N-Acetylglucosamin oder $\alpha$-DGlc NAcOMe ablöst und zu einem Impfstoff aufarbeitet.

18. Verwendung nach Anspruch 17, dadurch gekennzeichnet, daß die Merozoiten lysiert sind.

19. Verbindung der Formel 1 in Anspruch 1, worin $R^1$ Wasserstoff ist und $R^2$, $R^3$ und $R^4$ Wasserstoff oder alpha-L-Fucopyranosyl sind, wobei jedoch höchstens ein Rest alpha-L-Fucopyranosyl ist und die Verbindung ausgenommen ist, in der $R^3$ alpha-L-Fucopyranosyl ist und $R^1$, $R^2$ und $R^4$ Wasserstoff sind.

20. Verbindung der Formel 1 in Anspruch 1, worin $R^1$ $C_1$ bis $C_8$-Alkanyl, -Alkenyl oder -Alkinyl, jeweils verzweigt oder unverzweigt, oder ein alizyklischer oder aromatischer Kohlenwasserstoffrest, substituiert oder unsubstituiert, bevorzugt Benzyl oder substituiertes Benzyl, und $R^2$, $R^3$ und $R^4$ Wasserstoff oder alpha-L-Fucopyranosyl sind, wobei jedoch höchstens ein Rest alpha-L-Fucopyranosyl ist.

21. Verbindung der Formel 1 in Anspruch 1, worin $R^1$ $C_1$ bis $C_8$-Alkanyl, -Alkenyl oder -Alkinyl, jeweils verzweigt oder unverzweigt, oder ein alizyklischer oder aromatischer Kohlenwasserstoffrest, substituiert oder unsubstituiert, bevorzugt Benzyl oder substituiertes Benzyl, und $R^4$ 2,3,4-Tri-O-benzyl-alpha-L-Fucopyranosyl ist und $R^2$ und $R^3$ zusammen Alkyliden, bevorzugt Isopropyliden, sind,
oder $R^2$ 2,3,4-Tri-O-benzyl-alpha-L-Fucopyranosyl ist und $R^3$ und $R^4$ zusammen Alkyliden, bevorzugt Benzyliden, sind,

oder $R^3$ 2,3,4-Tri-O-benzyl-alpha-L-Fucopyranosyl ist und $R^2$ Benzyl, Benzyloxymethyl, Methyloxymethyl oder Allyl und $R^4$ Benzyl, Benzyloxymethyl oder Acyl, bevorzugt Acetyl, ist, wobei jedoch die Verbindung ausgenommen ist, in der $R^2$ Allyl oder Benzyl und $R^4$ Benzyl ist, sofern $R^1$ alpha-Benzyl ist.

22. Verbindung der Formel I in Anspruch 1, worin $R^1$ ein Spacer, der zur Anknüpfung an Proteine oder an aminierte Matrizes geeignet ist, die allgemeine Formel $-R-COR^5$ hat, eine Länge von 0,3 nm bis 3 nm besitzt und die Atome C, H, O, N, S und P enthalten kann, wobei eine Reaktionsmöglichkeit mit den übrigen in der Verbindung der Formel I enthaltenen Gruppen ausgeschlossen ist, und $R^2$, $R^3$ und $R^4$ Wasserstoff oder alpha-L-Fucopyranosyl sind, wobei jedoch höchstens ein Rest alpha-L-Fucopyranosyl ist, und wobei die Verbindung ausgenommen ist, in der $R^1$ $-(CH_2)_xCOR^5$ mit x = 3 - 17 und $R^5$ $C_1$-$C_8$-Alkoxy, O-Phenyl oder O-Benzyl ist, sofern die glycosidische Bindung zum Spacer beta ist.

23. Verbindung der Formel I in Anspruch 1, worin $R^1$ $-(CH_2)_nNHCO(CH_2)_mCOR^5$, $-(CH_2)_nCONH(CH_2)_mCOR^5$ oder $-(CH_2)_xCOR^5$ ist, wobei n = 1 bis 10, m = 1 bis 10, x = 3 bis 17, und $R^5$ -H, -OH oder $C_1$-$C_8$-Alkanoxy, -Alkenoxy oder -Alkinoxy, jeweils verzeigt oder unverzweigt, O-Phenyl, bevorzugt durch induktive Atome oder Gruppen, wie $NO_2$, substituiert, O-Benzyl, substituiert oder unsubstituiert, oder $-NH_2$, $-NHNH_2$ oder $-N_3$ ist, und $R^2$, $R^3$ und $R^4$ Wasserstoff oder alpha-L-Fucopyranosyl sind, wobei jedoch höchstens ein Rest alpha-L-Fucopyranosyl ist, und wobei die Verbindung ausgenommen ist, in der $R^1$ $-(CH_2)_xCOR^5$ mit x = 3-17 und $R^5$ $C_1$- $C_8$-Alkoxy, O-Phenyl oder O-Benzyl ist, sofern die glykosidische Bindung zum Spacer beta ist.

24. Verbindung der Formel I in Anspruch 1, worin $R^1$ ein Spacer, der zur Anknüpfung an Proteine oder an aminierte Matrizes geeignet ist, die allgemeine Formel $-R-COR^5$ hat, eine Länge von 0,3 nm bis 3 nm besitzt und die Atome C, H, O, N, S und P enthalten kann, wobei eine Reaktionsmöglichkeit mit den übrigen in der Verbindung der Formel I enthaltenen Gruppen ausgeschlossen ist, und $R^4$ Wasserstoff oder 2,3,4-Tri-O-benzyl-alpha-L-fucopyranosyl ist und $R^2$ und $R^3$ zusammen Alkyliden, bevorzugt Isopropyliden, sind.

25. Verbindung der Formel I in Anspruch 1, worin $R^1$ ein Spacer, der zur Anknüpfung an Proteine oder an aminierte Matrizes geeignet ist, die allgemeine Formel $-R-COR^5$ hat, eine Länge von 0,3 nm bis 3 nm besitzt und die Atome C, H, O, N, S und P enthalten kann, wobei eine Reaktionsmöglichkeit mit den übrigen in der Verbindung der Formel I enthaltenen Gruppen ausgeschlossen ist, und $R^2$ Wasserstoff oder 2,3,4-Tri-O-benzyl-alpha-fucopyranosyl ist und $R^3$ und $R^4$ zusammen Alkyliden, bevorzugt Benzyliden, sind.

26. Verbindung der Formel I in Anspruch 1, worin $R^1$ ein Spacer, der zur Anknüpfung an Proteine oder an aminierte Matrizes geeignet ist, die allgemeine Formel $-R-COR^5$ hat, eine Länge von 0,3 nm bis 3 nm besitzt und die Atome C, H, O, N, S und P enthalten kann, wobei eine Reaktionsmöglichkeit mit den übrigen in der Verbindung der Formel I enthaltenen Gruppen ausgeschlossen ist, und $R^3$ Wasserstoff oder 2,3,4-Tri-O-benzyl-alpha-L-fucopyranosyl ist und $R^2$ Benzyl, Benzyloxymethyl, Methyloxymethyl oder Allyl und $R^4$ Benzyl, Benzyloxymethyl oder Acyl, bevorzugt Acetyl, ist.

27. Verbindung der Formel I in Anspruch 1, worin $R^1$ $-(CH_2)NHCO(CH_2)_mCOR^5$, $-(CH_2)_nCONH(CH_2)_mCOR^5$

oder $-(CH_2)_xCOR^5$ ist, wobei n = 1 bis 10, m = 1 bis 10, x = 3 bis 17, und $R^5$ -H, -OH oder $C_1$-$C_8$-Alkanoxy, -Alkenoxy oder-Alkinoxy, jeweils verzeigt oder unverzweigt, O-Phenyl, bevorzugt durch induktive Atome oder Gruppen, wie $NO_2$, substituiert, O-Benzyl, substituiert oder unsubstituiert, oder $-NH_2$, $-NHNH_2$ oder $-N_3$ ist, und $R^4$ Wasserstoff oder 2,3,4-Tri-O-benzyl-alpha-L-fucopyranosyl ist und $R^2$ und $R^3$ zusammen Alkyliden, bevorzugt Isopropyliden, sind.

28. Verbindung der Formel I in Anspruch 1, worin $R^1$ $-(CH_2)_nNHCO(CH_2)_mCOR^5$, $-(CH_2)_nCONH(CH_2)_mCOR^5$ oder $-(CH_2)_xCOR^5$ ist, wobei n = 1 bis 10, m = 1 bis 10, x = 3 bis 17, und $R^5$ -H, OH oder $C_1$-$C_8$-Alkanoxy, -Alkenoxy oder-Alkinoxy, jeweils verzeigt oder unverzweigt, O-Phenyl, bevorzugt durch induktive Atome oder Gruppen, wie $NO_2$, substituiert, O-Benzyl, substituiert oder unsubstituiert, oder $-NH_2$, $-NHNH_2$ oder $-N_3$ ist, und $R^2$ Wasserstoff oder 2,3,4-Tri-O-benzyl-alpha-L-fucopyranosyl ist und $R^3$ und $R^4$ zusammen Alkyliden, bevorzugt Benzyliden, sind.

29. Verbindung der Formel I in Anspruch 1, worin $R^1$ $-(CH_2)_nNHCO(CH_2)_mCOR^5$, $-(CH_2)_nCONH(CH_2)_mCOR^5$ oder $-(CH_2)_xCOR^5$ ist, wobei n = 1 bis 10, m = 1 bis 10, x = 3 bis 17, und $R^5$ -H, -OH oder $C_1$-$C_8$-Alkanoxy, -Alkenoxy oder -Alkinoxy, jeweils verzeigt oder unverzweigt, O-Phenyl, bevorzugt durch induktive Atome oder Gruppen, wie $NO_2$, substituiert, O-Benzyl, substituiert oder unsubstituiert, oder $-NH_2$, $-NHNH_2$ oder $-N_3$ ist, und $R^3$ Wasserstoff oder 2,3,4-Tri-O-benzyl-alpha-L-fucopyranosyl ist und $R^2$ Benzyl, Benzyloxymethyl, Methyloxymethyl oder Allyl und $R^4$ Benzyl, Benzyloxymethyl oder Acyl, bevorzugt Acetyl, ist.

30. Verbindung der Formel I in Anspruch 1, worin $R^1$ ein Spacer, gebunden an einen natürlichen oder synthetischen Träger, bevorzugt an eine aminierte Matrix, ist und $R^2$, $R^3$ und $R^4$ Wasserstoff oder alpha-L-Fucopyranosyl sind, wobei jedoch höchstens ein Rest alpha-L-Fucopyranosyl ist, wobei die Verbindung ausgenommen ist, in der der Spacer $-(CH_2)_xCOR^5$ ist, worin x = 3 - 17 und $R^5$ ein Träger ist, sofern die glycosidische Bindung zum Spacer beta ist.

31. Verfahren zur Herstellung einer Verbindung der Formel I in Anspruch 1, dadurch gekennzeichnet, daß man eine Verbindung der Formel I, in der $R^1$ ein $C_1$ bis $C_8$-Alkanyl, -Alkenyl oder -Alkinyl, jeweils verzweigt oder unverzweigt, oder ein alizyklischer oder aromatischer Kohlenwasserstoff-Rest, substituiert oder unsubstituiert, bevorzugt Benzyl oder substituiertes Benzyl oder $-(CH_2)_nNHCO(CH_2)m$ $COR^5$, $-(CH_2)_nCONH(CH_2)_mCOR^5$ oder $-(CH_2)_xCOR^5$ ist, wobei n = 1 bis 10, m = 1 bis 10, x = 3 bis 17 und $R^5$ $C_1$-$C_8$-Alkanoxy, -Alkenoxy oder -Alkinoxy, jeweils verzweigt oder unverzweigt, O-Phenyl, bevorzugt durch induktive Atome oder Gruppen, wie $NO_2$, substituiert, O-Benzyl, substituiert oder unsubstituiert, ist und $R^4$ Wasserstoff und $R^2$ und $R^3$, zusammen mit dem Brücken-C-Atom, Alkyliden oder $R^2$ Wasserstoff und $R^3$ und $R^4$, zusammen mit dem Brücken-C-Atom, Benzyliden oder Alkyliden oder $R^3$ Wasserstoff, $R^2$ Benzyl, Benzyloxymethyl, Methyloxymethyl oder Allyl und $R^4$ Benzyl, Benzyloxymethyl oder Acyl ist, mit 2,3,4-Tri-O-benzyl-alpha-L-fucopyranosylbromid oder 1-O-(N-methyl)-acetimidyl-2,3,4-tri-O-benzyl-ß-L-fucopyranose reagieren läßt zu einer Verbindung der Formel I in Anspruch 1, worin $R^2$, $R^3$ oder $R^4$

2,3,4-Tri-O-benzyl-alpha-L-fucopyranosyl ist und jeweils $R^3$ und $R^4$, $R^2$ und $R^4$ oder $R^2$ und $R^3$ sowie $R^1$ die in diesem Anspruch angegebene Bedeutung haben.

32. Mittel zur Chemotherapie und Chemoprophylaxe der Malaria, dadurch gekennzeichnet, daß es eine Verbindung der Formel I nach Anspruch 1 und einen pharmazeutisch unbedenklichen Hilfs- und/oder Zusatzstoff enthält.

33. Verwendung einer Verbindung der Formel I in Anspruch 1, worin $R^1$ ein Spacer ist, der an einen festen Träger, bevorzugt an eine aminierte Matrix, gebunden ist, und $R^2$ bis $R^4$ Wasserstoff oder alpha-L-fucopyranosyl sind, wobei jedoch höchstens ein Rest alpha-L-fucopyranosyl ist, in einem Verfahren zur Herstellung eines Impfstoffes gegen die Malaria, dadurch gekennzeichnet, daß man diese Verbindung mit intakten Merozoiten oder einem Merozoiten-Lysat inkubiert, die intakten Merozoiten lysiert, den Träger wäscht, das gebundene Produkt bei einem pH von 2.5 bis 5 oder von 8 bis 11 oder durch chaotrope Änderung oder durch Zusatz von N-Acetylglucosamin oder $\alpha$-DGlcNAcOMe eluiert, das eluierte Produkt gegebenenfalls weiter reinigt und zu einem Impfstoff zur aktiven Immunisierung gegen Malaria aufarbeitet.

0096293

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

Nummer der Anmeldung

EP 83 10 5147

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | FR - A - 2 016 182 (ROTTA RESEARCH)<br>* Seite 88 *<br>-- | 24 | C 07 H 13/04<br>C 07 H 15/04<br>C 07 H 3/04<br>A 61 K 31/70<br>A 61 K 39/015<br>A 61 K 39/00 |
| X,Y | EP - A - 0 044 188 (CHEMBIOMED LTD.)<br>* Seiten 25-32 *<br>-- | 1-14, 16-33 | |
| X,Y | FR - A - 2 332 998 (CANADIAN PATENTS AND DEVELOPMENT LIMITED)<br>* Seiten 33-37 *<br>-- | 1-14, 16-33 | |
| P,X, Y | EP - A - 0 060 999 (BEHRINGWERKE A.G.)<br>* Seiten 1-2 * -- ./. | 1-14, 16-33 | **RECHERCHIERTE SACHGEBIETE (Int. Cl. 3)**<br>C 07 H 13/00<br>C 07 H 15/00<br>C 07 H 3/00<br>A 61 K 31/00<br>A 61 K 39/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 16-33

Unvollständig recherchierte Patentansprüche: 1-14

Nicht recherchierte Patentansprüche: 15

Grund für die Beschränkung der Recherche:

Verfahren zur chirurgischen oder therapeutischen Behandlung des menschlichen oder tierischen Körpers (siehe Art. 52(4) des Europäischen Patentübereinkommens).

| Recherchenort | Abschlußdatum der Recherche | Prüfer |
|---|---|---|
| Den Haag | 21-08-1983 | VERHULST |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

Europäisches Patentamt

**EUROPÄISCHER TEILRECHERCHENBERICHT**

0096293
Nummer der Anmeldung

-2- EP 83 10 5147

| EINSCHLÄGIGE DOKUMENTE | | | KLASSIFIKATION DER ANMELDUNG (Int. Cl. ) |
|---|---|---|---|
| Kategorie | Kennzeichnung des Dokuments mit Angabe, soweit erforderlich der maßgeblichen Teile | betrifft Anspruch | |
| Y | NATURE, Band. 289, Nr. 5793, Januar 1981, Macmillan Journals Ltd. PARIS (FR) W.A. SIDDIQUI et al.: "Use of a synthetic adjuvant in an effective vaccination of monkeys against malaria", Seiten 64-66.<br>* Seite 64 *<br>---- | 32,33 | |
| | | | RECHERCHIERTE SACHGEBIETE (Int. Cl.³) |

EPA Form 1505.3 06.78